(19) Europäisches Patentamt
European Patent Office
Office européen des brevets

(11) Veröffentlichungsnummer: **0 011 281 B1**

# (12) EUROPÄISCHE PATENTSCHRIFT

(45) Veröffentlichungstag der Patentschrift: **10.02.82**

(51) Int. Cl.³: **C 07 C 45/43**, C 07 C 47/542, C 07 C 47/55, C 07 C 21/24

(21) Anmeldenummer: **79104477.9**

(22) Anmeldetag: **13.11.79**

## (54) Verfahren zur Herstellung von p-tert.-Butylbenzaldehyd und dessen am Kern durch Halogen substituierten Derivaten.

(30) Priorität: **16.11.78 DE 2849692**
**22.03.79 DE 2911237**

(43) Veröffentlichungstag der Anmeldung: **28.05.80 Patentblatt 80/11**

(45) Bekanntmachung des Hinweises auf die Patenterteilung: **10.02.82 Patentblatt 82/6**

(84) Benannte Vertragsstaaten: **AT BE CH DE FR GB IT NL**

(56) Entgegenhaltungen:
**FR-A-2163741**
**GB-A-816253**

**CHEMICAL ABSTRACTS, Band 87, 1977, Zusammenfassung Nr. 84685j, Columbus, Ohio, US**

(73) Patentinhaber: **HOECHST AKTIENGESELLSCHAFT, Postfach 80 03 20, D-6230 Frankfurt/Main 80 (DE)**

(72) Erfinder: **Landauer, Franz, Dr., Liederbacher Strasse 7, D-6230 Frankfurt am Main 80 (DE)**
Erfinder: **Schaeffer, Georg, Dr., Herderstrasse 58, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

Verfahren zur Herstellung von p-tert.-Butylbenzaldehyd und dessen am Kern durch Halogen substituierten Derivaten.

p-tert.-Butylbenzaldehyd und dessen am Kern durch halogen substituierte Derivate sind wertvolle Zwischenprodukte für die Herstellung insbesondere von Pharmazeutika, Pflanzenschutzmitteln, Farbstoffen und Riechstoffen.

Die Herstellung des p-tert.-Butylbenzaldehyds und dessen Derivate erfolgt durchweg nach den für die Darstellung aromatischer Aldehyde üblichen Methoden.

So kann man beispielsweise durch Umsetzung von tert.-Butylbenzol mit CO/HCl in Gegenwart von CuCl zu p-tert.-Butylbenzaldehyd gelangen. Der Nachteil dieser Methode besteht jedoch insbesondere in der Notwendigkeit der Verwendung eines Cu-Salzes, wodurch dann eine besondere Abwasserreinigung erforderlich wird.

Ein anderes bekanntes Verfahren, bei dem ebenfalls ein Cu-Salz — $Cu(NO_3)_2$ — zum Einsatz kommt, geht aus von p-tert.-Butyltoluol, welches durch Umsetzung mit einer etwa äquivalenten Menge Brom zunächst in p-tert.-Butylbenzylformid überführt wird; daraus wird dann durch längeres Kochen mit einer $Cu(NO_3)_2$-Lösung p-tert.-Butylbenzaldehyd mit einer Ausbeute von etwa 42% d.Th. gewonnen („J. Chem. Soc.", 1935, S. 1848).

Bei einem weiteren Verfahren zur Überführung des p-tert.-Butylbenzylbromids in p-tert.-Butylbenzaldehyd wurde die $Cu(NO_3)_2$-Lösung der letztgenannten Methode durch eine Lösung von Urotropin in wässrigem Äthanol ersetzt („Sommelet-Reaktion", *in* „J. Chem. Soc.", 1940, S. 702). Da bei dieser Reaktion aus dem Urotropin ein Gemisch von Methylamin, Ammoniak und Formaldehyd anfällt, dessen Beseitigung aus Umweltschutzgründen notwendig ist, ist auch hier — ebenso wie bei den beiden vorgenannten Cu-Salz-Methoden — eine aufwendige und kostspielige Abwasserreinigung nötig. Ausserdem erfordern alle vorerwähnten Methoden einen erheblichen Energieverbrauch und liefern nur unzureichende Raum-Zeit-Ausbeuten.

Die Anwendung einer weiteren für die Herstellung aromatischer Aldehyde bekannten Methode (Verseifung von Benzalchloriden mit Wasser, vergleiche z.B. DE-OS Nr. 2044832) auf die Herstellung des p-tert.-Butylbenzaldehyds erwies sich, wie eigene Versuche zeigten, als wenig vorteilhaft, weil bei der Herstellung des hier erforderlichen p-tert.-Butylbenzalchlorids durch radikalische Chlorierung von p-tert.-Butyltoluol Produkte erhalten werden, deren organisch gebundenes Chlor nur zum Teil unter den üblichen Bedingungen durch alkalische Hydrolyse wieder abgespalten werden kann (vgl. Houben-Weyl, „Methoden der Org. Chemie", Band II, S. 233, Stuttgart 1953), was auf eine nicht unbeträchtliche Kernchlorierung hindeutet. Dies ist — wenn man am Ende reinen p-tert.-Butylbenzaldehyd haben will — natürlich ziemlich unerwünscht. In manchen Fällen werden zwar für die Herstellung von Pharmazeutika, Pflanzenschutzmitteln, Farbstoffen, Riechstoffen usw. auch am Kern durch Chlor substituierte Derivate des p-tert.-Butylbenzaldehyds benötigt; das Chlor sollte jedoch gezielt, wie auch die anderen Halogene (F, Br, J), vor der Seitenkettenhalogenierung eingeführt werden.

Es bestand daher die Aufgabe, ein Verfahren zur Herstellung von p-tert.-Butylbenzaldehyd sowie von dessen am Kern durch Halogen substituierten Derivaten zu finden, welches die Nachteile der bekannten Methoden nicht aufweist, welches also keine kostspielige Abwasserreinigung erfordert, gute Ausbeuten an dem gewünschten Produkt und keine in unerwünschter Weise substituierten Nebenprodukte liefert und auch sonst in jeder Beziehung wirtschaftlich ist.

Diese Aufgabe konnte erfindungsgemäss in einfacher und ausgezeichneter Weise dadurch gelöst werden, dass man — ausgehend von p-tert.-Butyltoluol und dessen am Kern durch Halogen substituierten Derivaten — durch Seitenkettenbromierung zuerst das entsprechende Benzalbromid herstellt und dieses dann zum gewünschten Aldehyd verseift.

Erfindungsgegenstand ist daher ein Verfahren zur Herstellung von p-tert.-Butylbenzaldehyd und dessen am Kern durch Halogen substituierten Derivaten, das dadurch gekennzeichnet ist, dass man

a) p-tert.-Butyltoluol und dessen am Kern durch Halogen substituierte Derivate mit etwa 2 mol Brom/mol organisches Ausgangsmaterial bei Temperaturen von etwa 40 bis 200, vorzugsweise etwa 40 bis 120°C, gegebenenfalls unter der Einwirkung energiereicher Strahlung oder in Gegenwart von Radikalbildnern umsetzt und

b) das dabei gebildete p-tert.-Butylbenzalbromid und dessen am Kern durch Halogen substituierte Derivate gegebenenfalls nach deren Isolierung bei erhöhter Temperatur, gegebenenfalls in Gegenwart von üblichen Verseifungskatalysatoren, mit Wasser verseift.

Als Ausgangsmaterialien für das Verfahren dienen p-tert.-Butyltoluol und dessen am Kern durch Halogen substituierte — insbesondere monosubstituierte — Derivate. Die monosubstituierten Derivate können durch folgende allgemeine Formel (I) wiedergegeben werden:

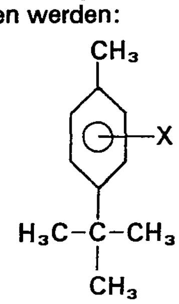

(I)

worin X = F, Cl, Br, J, insbesondere F, Cl, Br.

Das jeweilige Ausgangsprodukt wird dann mit etwa 2 (1,8 bis 2,2, insbesondere 1,9 bis 2,1) mol Brom/mol Ausgangsmaterial bei den angegebenen Temperaturen — etwa 40 bis 200, vorzugsweise etwa 40 bis 120°C — gegebenenfalls unter

der Einwirkung energiereicher Strahlung oder in Gegenwart von Radikalbildnern umgesetzt. Als energiereiche Strahlung kommt vorzugsweise UV-Licht infrage.

Geeignete Radikalbildner sind die für Seitenkettenchlorierungen üblichen organischen Peroxide, Azoisobutyronitril usw.

Die energiereiche Strahlung oder die Gegenwart von Radikalbildnern sind für das Gelingen der Reaktion zwar nicht unbedingt erforderlich, jedoch in erheblichem Mass reaktionsbeschleunigend und daher vorteilhaft.

Das Brom kann bei dieser Reaktion entweder flüssig zugetropft oder gasförmig (nach dem Verdampfen) eingeleitet werden, wobei im letzteren Fall auch ein Inertgas (Stickstoff, Argon usw.) zugesetzt werden kann.

Die Bromierung kann sowohl ohne als auch in einem geeigneten Lösungsmittel durchgeführt werden, wobei als Lösungsmittel inerte — insbesondere halogenierte — Kohlenwasserstoffe wie z.B. $CCl_4$ oder o-Dichlorbenzol in Betracht kommen.

Weiterhin ist die Durchführung der Reaktion drucklos oder unter erhöhtem Druck sowie sowohl diskontinuierlich als auch kontinuierlich möglich.

Das bei dieser Bromierung gebildete p-tert.-Butylbenzalbromid sowie — wenn die am Kern durch Halogen substituierten Derivate des p-tert.-Butyltoluols als Ausgangsstoffe verwendet wurden — die entsprechenden am Kern durch Halogen substituierten Derivate besitzen keine unerwünschten Bromsubstituenten am aromatischen Kern und sind neue Verbindungen. Sie besitzen die allgemeine Formel (II):

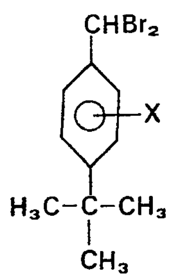

(II)

worin X = H, F, Cl, Br, J,
vorzugsweise = H, F, Cl, Br,
insbesondere = H.

Die unter diese Formel fallenden Verbindungen sind also:

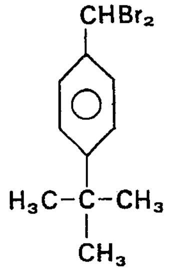
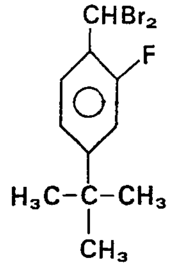
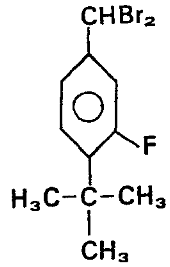
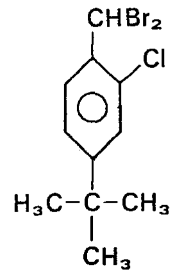
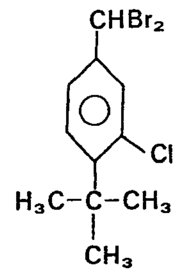
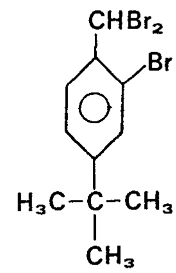
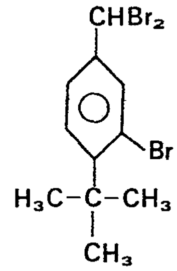
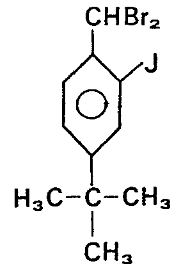
und
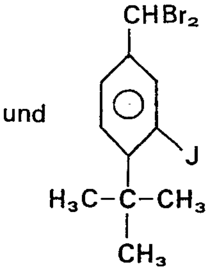

Für die Weiterverarbeitung zum entsprechenden Benzaldehyd können die Verbindungen (II) entweder isoliert und gereinigt (z.B. durch Umkristallisation) oder ohne Isolierung verseift werden.

Die Verseifung erfolgt nach den für die Verseifung von Benzalhalogeniden üblichen Methoden bei erhöhter Temperatur — vorzugsweise bei Temperaturen von etwa 60 bis 150, insbesondere etwa 80 bis 120°C — mit Wasser, gegebenenfalls in Gegenwart von üblichen Verseifungskatalysatoren, z.B. von Metallhalogeniden wie $FeCl_3$, $ZnBr_2$ usw., oder auch von $H_2SO_4$ und dgl. Vorzugsweise wird pro Mol Benzalbromid (II) 1 mol Wasser verwendet; es kann aber auch ein Überschuss an Wasser eingesetzt werden, wobei jedoch dann eine zusätzliche Abtrennung der wässrigen Phase durchzuführen ist. In der Regel wird zu vorgelegtem Benzalbromid (+ Verseifungskatalysator) Wasser in dem Mass zugegeben, in welchem sich Bromwasserstoff bildet und anschliessend absorbiert werden kann.

Die Verseifung kann im übrigen z.B. sowohl drucklos als auch under erhöhtem Druck durchgeführt werden. Man kann auch inerte Lösungsmittel wie z.B. Kohlenwasserstoffe oder Chlorkohlenwasserstoffe zusetzen. Ferner ist noch etwa die Verwendung von Emulgatoren möglich, die eine gute Vermischung der organischen Phase mit dem zugegebenen Wasser bewirken.

Die Absorption des Bromwasserstoffs erfolgt zweckmässig in einer üblichen Absorptionsanlage; er kann dann direkt für weitere chemische Reaktionen verwendet oder verkauft werden.

Nach dem erfindungsgemässen Verfahren werden p-tert.-Butylbenzaldehyd und dessen am Kern durch Halogen substituierte Derivate. Die Produkte besitzen die allgemeine Formel (III):

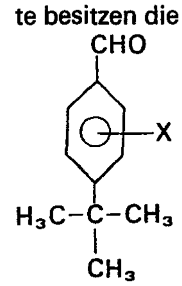

(III)

worin X = H, F, Cl, Br, J,
vorzugsweise = H, F, Cl, Br,
insbesondere = H.

in ausgezeichneten Ausbeuten enthalten sind. Die Produkte besitzen — ausser den schon in den Ausgangsmaterialien vorhandenen Kernsubstituenten — keine unerwünschten (Brom-)Substituenten mehr. Diese Tatsache ist ausserordentlich überraschend, da Halogenierungen mit Chlor und Brom normalerweise immer gleichartig verlaufen, in diesem Fall, bei Verwendung von Chlor, aber durch Seitenkettenchlorierung von p-tert.-Butyltoluol eine beträchtliche Bildung von Nebenprodukten nicht zu vermeiden ist. Im Gegensatz dazu findet bei der Bromierung des p-tert.-Butyltoluols (und dessen entsprechender Substitutionsprodukte) keine oder so gut wie keine unerwünschte Kernhalogenierung und darüberhinaus auch keine Bromierung der tert.-Butylgruppe statt. Die hohe Selektivität und Ausbeute des Verfahrens sowie die Tatsache, dass — im Gegensatz zu etlichen Verfahren des Standes der Technik — keinerlei besondere Abwasseraufbereitung erforderlich ist, stellen einen erheblichen Fortschritt dar.

Trotz des günstigen und selektiven Verlaufs der Reaktion kommt es jedoch — auch wenn man ziemlich genau die stöchiometrische Menge (2 mol) Brom/mol organisches Ausgangsmaterial (p-tert.-Butyltoluol oder dessen entsprechendes, am Kern durch Halogen substituiertes Derivat) verwendet — vor, dass bei der Bromierung oft noch eine geringe Menge Benzylbromid sowie auch Benzotribromid gebildet wird. Bei der anschliessenden Verseifung, bei welcher aus dem Hauptbromierungsprodukt (Benzalbromid) der gewünschte Aldehyd entsteht, wird das Benzylbromid-Nebenprodukt nicht mitverseift und bleibt somit unverändert in dem Reaktionsgemisch erhalten. Das Benzotribromid-Nebenprodukt wird dagegen zur entsprechenden Benzoesäure verseift und kann etwa durch Extraktion mit mildem Alkali leicht entfernt werden.

Die Reinigung des Aldehyds von dem in geringer Menge beigemengten entsprechenden Benzylbromid, das unter den angewandten Verseifungsbedingungen nicht verseift, kann praktisch nur durch mehrmalige aufwendige Rektifikation im Vakuum erreicht werden. Für viele Verwendungszwecke des Aldehyds sind geringe Mengen an Benzylbromid-Verunreinigung nicht störend; für andere Verwendungszwecke — wenn der Aldehyd beispielsweise in der Riechstoffindustrie oder als Zwischenprodukt etwa für die Herstellung von Pharmazeutika eingesetzt werden soll — ist jedoch eine praktisch 100%ige Reinheit und damit die restlose Entfernung des im Aldehyd vorhandenen Benzylbromids notwendig. Ausserdem verfärbt sich p-tert.-Butylbenzaldehyd, der noch geringe Mengen an p-tert.-Butylbenzylbromid enthält, beim Stehen schneller als vollkommen reiner Aldehyd.

In einer besonderen Ausführungsform des erfindungsgemässen Vefahrens gelingt es jedoch auch, ohne grösseren Aufwand einen reineren p-tert.-Butylbenzaldehyd und reinere entsprechende am Kern durch Halogen substituierte Derivate, insbesondere ohne beigemengtes entsprechendes Benzylbromid, zu erhalten: wenn man nämlich während oder nach der Verseifungsstufe b noch eine geringe Menge an Formaldehyd $CH_2O$+Ammoniak $NH_3$ oder von Hexamethylentetramin in wässriger Lösung zusetzt und erhitzt.

Dadurch wird das vorhandene Benzylbromid-Nebenprodukt in den entsprechenden Aldehyd überführt (Sommelet-Reaktion). Wenn die genannten Stoffe $CH_2O+NH_3$ oder Hexamethylentramin während der Verseifung zugesetzt werden, ist es zweckmässig, den Reaktionsansatz in Gegenwart dieser Zusatzstoffe noch eine bis zu mehrere Stunden bei den für die Verseifung angewandten Temperaturen (vorzugsweise etwa 60 bis 150, insbesondere etwa 80 bis 120°C) zu belassen. Da die Verseifung in wässriger Lösung durchgeführt wird, ist normalerweise auch das für die Überführung des entsprechenden Benzylbromids in den Aldehyd nötige Wasser bereits vorhanden. Bei von der Verseifung her nicht ausreichender Wassermenge ist noch Wasser zuzusetzen.

Vorzugsweise wird die Überführung des entsprechenden Benzylbromids in Aldehyd nach der Verseifung durchgeführt. Dies kann sowohl in dem zu Ende reagierten Verseifungsansatz oder auch nach Isolierung des (nebenprodukthaltigen) Benzaldehyds geschehen. In jedem Falle ist wichtig, dass $CH_2O+NH_3$ oder Hexamethylentetramin in wässriger Lösung einige Zeit — im allgemeinen etwa 1 bis mehrere Stunden — bei erhöhter Temperatur, vorzugsweise bei etwa 60 bis 150, insbesondere etwa 80 bis 120°C, auf das in den Aldehyd umzuwandelnde Benzylbromid einwirken können.

Die Menge des eingesetzten $CH_2O+NH_3$ kann der (etwa gaschromatographisch bestimmten) Menge des Benzylbromid-Nebenprodukts etwa äquimolar sein (1 mol $CH_2$+1 mol $NH_3$ auf 1 mol Benzylbromid); es ist jedoch bevorzugt, $CH_2O+NH_3$ im Überschuss zuzugeben, wobei pro mol p-tert.-Butylbenzylbromid oder dessen entsprechend kernhalogensubstituiertem Derivat etwa 1,1 bis 4 mol, insbesondere etwa 1,1 bis 2 mol $CH_2O$+die gleiche Molzahl $NH_3$ eingesetzt werden. Anstelle dieser Mengen $CH_2O+NH_3$ ist selbstverständlich auch die Verwendung von äquivalenten Mengen Hexamethylentetramin (Urotropin) möglich, was die bevorzugte Ausführungsform darstellt.

Die Durchführung der während oder nach der Verseifungsstufe des Verfahrens betriebenen Sommelet-Reaktion kann schliesslich sowohl unter Normal- als auch unter Überdruck erfolgen.

Der Reaktionsansatz wird im Falle dieser Verfahrensvariante nach beendeter Reaktion zweckmässig so aufgearbeitet, dass zur Entfernung des Nebenprodukts p-tert.-Butylbenzoesäure (bzw. dem entsprechenden kernhalogensubstituierten Derivat) zunächst mit mildem Alkali extrahiert wird; aus dem verbliebenen Teil wird der reine Aldehyd dann durch Vakuumdestillation gewonnen.

Überraschenderweise kann bei der Durchführung der Sommelet-Reaktion gemäss der hier beschriebenen Verfahrensvariante auf die bei der

Sommelet-Reaktion sonst verwendeten organischen Löse- und Verdünnungsmittel (Alkohl, $CHCl_3$, Essigsäure usw., siehe Weygand/Hilgetag, „Organisch-Chemische Experimentierkunst", Verlag Johann Ambrosium Barth Leipzig 1970, Seite 346) verzichtet werden. Dies bedeutet einen erheblichen Vorteil, da hierdurch keine aufwendigen Auftrennungs- und Wiedergewinnungsanlagen notwendig sind.

Die Verfahrensvariante ermöglicht in technisch einfacher Weise, reinen p-tert.-Butylbenzaldehyd und dessen am Kern durch Halogen substituierte Derivate ohne aufwendige Rektifikationsapparaturen zu erhalten. Gegenüber der reinen Sommelet-Reaktion, ausgehend nur von p-tert.-Butylbenzylbromid und dessen am Kern durch Halogen substituierten Derivaten hat die Verfahrensvariante den Vorteil, dass nur relativ wenig $CH_2O+NH_3$ oder Hexamethylentetramin verwendet werden muss, so dass der eingangs beschriebene Hauptnachteil dieser Reaktion (stark mit $CH_2O+NH_3$ bzw. $NH_4Br$ verunreinigtes Produktionsabwasser) hier praktisch nicht ins Gewicht fällt.

An der Umweltfreundlichkeit und Wirtschaftlichkeit des Verfahrens ändert sich daher praktisch nichts. Wegen der Umwandlung des Benzylbromid-Nebenprodukts in den gewünschten Aldehyd liegt die Aldehydausbeute über derjenigen, die ohne die beschriebene besondere Verfahrensvariante erhalten wird.

Die Erfindung wird nun anhand der folgenden Beispiele näher erläutert. Auf die (Erfindungs-) Beispiele folgt dann ein Vergleichsbeispiel, bei welchem als Halogenierungsmittel für das p-tert.-Butyltoluol anstelle des Broms Chlor verwendet wurde.

*Beispiel 1:*

In einem 1 l Vierhalskolben mit Rührer, Thermometer, Tropftrichter und Rückflusskühler, der mit einer (mit Wasser gefüllten) Absorptionsvorrichtung für Bromwasserstoff verbunden ist, werden 296 g p-tert.-Butyltoluol (2 mol) auf 100 bis 110°C erhitzt. Unter Bestrahlung mit UV-Licht tropft man dann im Verlauf von 3,5 h 656 g Brom (4,1 mol) zu. Anschliessend wird mit Stickstoff der noch im Reaktionsprodukt befindliche Bromwasserstoff ausgeblasen.

Rohausbeute p-tert.-Butylbenzalbromid: 606 g (=98,7% d.Th.).

gef.: Gesamt-Brom: 53,3%

verseifbares Brom: 53,4%; ber.: 52,3%.

Eine gleich gute Ausbeute wird erhalten, wenn das Brom gasförmig eingeleitet wird.

Fp. des reinen p-tert.-Butylbenzalbromids: 44°C (umkristallisiert aus Äthanol).

Zu dem rohen p-tert.-Butylbenzalbromid gibt man 0,6 g $ZnCl_2$ und 0,3 g Wasser, erhitzt auf 110°C und lässt im Verlauf von 4 h 36 g Wasser zutropfen, wobei die Reaktionstemperatur allmählich auf 90 bis 100°C gesenkt wird. Der bei der Verseifung entstehende Bromwasserstoff wird in einer nachgeschalteten Apparatur in Wasser aufgefangen.

Nach Zugabe des Wassers wird 30 min nachgerührt und der restliche Bromwasserstoff mit Stickstoff ausgeblasen. Zurückerhaltener Bromwasserstoff: 316 g (= 97,5% d.Th.).

Der rohe p-tert.-Butylbenzaldehyd wird anschliessend im Vakuum ($Kp_3$: 90°C) destilliert.

Ausbeute: 299 g=92,5% d.Th. (=1,85 mol).

*Beispiel 2:*

In gleicher Weise wie in Beispiel 1 wurde ausgehend von 2 mol p-tert.-Butyltoluol und 4,0 mol Brom nochmals roher p-tert.-Butylbenzaldehyd hergestellt und das diesem beigemengte p-tert.-Butylbenzylbromid nach der erfindungsgemässen Verfahrensvariante in Aldehyd überführt.

Der rohe p-tert.-Butylbenzaldehyd enthielt gemäss GC-Analyse noch 7,3% p-tert.-Butylbenzylbromid.

Zu dem rohen Aldehydgemisch wird dann eine Lösung von 19 g Hexamethylentetramin in 40 g $H_2O$ gegeben und 2 h bei 100 bis 110°C gerührt.

In einer Probe des Reaktionsgemisches wurden dann nur noch ein Gehalt an p-tert.-Butylbenzylbromid von $<0{,}3\%$ gefunden (CG-Analyse).

Durch anschliessendes Ausrühren mit einer wässrigen Sodalösung wurde die p-tert.-Butylbenzoesäure extrahiert. Nach Ansäuern der abgetrennten wässrigen Lösung mit Salzsäure wird die p-tert.-Butylbenzoesäure ausgefällt. Es wurden 12 g Säure erhalten.

Der Rohaldehyd wird nun im Vakuum destilliert.

$Kp._{10}$: 110°C

Ausbeute: 310 g=95,6% d.Th.

*Beispiel 3:*

In einem 250 ml Glaskolben wurden 100 g =0,548 mol 1-Methyl-2-chlor-4-tertbutylbenzol unter UV-Bestrahlung und unter Rühren mit 175,5g=1,097 mol Brom folgendermassen zur Reaktion gebracht:

Bei einer Reaktionstemperatur von 130°C wurden innerhalb 60 min 100 g=0,625 mol flüssiges Brom eingetropft. Dann wurde die Reaktionstemperatur auf 180°C erhöht und innerhalb 30 min wurden weitere 75,5 g=0,472 mol Brom eingetropft. Bei 180°C wurde dann noch 30 min lang nachgerührt. Dann wurde das Reaktionsgemisch auf 20°C abgekühlt und von darin gelösten geringen Mengen Bromwasserstoff und Brom mittels Durchleiten eines Stickstoffstromes befreit.

Nach dem Ausblasen wurden 186 g einer rohen Lösung erhalten, die zu 85% aus 2-Chlor-4-tertbutylbenzalbromid bestand. Die Identität des Produktes wurde durch Massenspektren und NMR-Spektren bestätigt, die an dem destillierten Produkt ($Kp._{0,2}$: 115°C) aufgenommen wurden.

*Vergleichsbeispiel:*

In genau der gleichen Apparatur und unter den gleichen Bedingungen wir in (Erfindungs-)Beispiel 1 wurden 296 g p-tert.-Butyltoluol (2 mol) durch Einleiten von 284 g (ca. 4,1 mol) Chlor im Verlauf von 3,5 h chloriert. Nach dem Ausblasen des Chlorwasserstoffs betrug die Rohausbeute 415 g; darin wurden gefunden:

Gesamtchlor: 31,05%

verseifbares Chlor: 20,85% (Bestimmung: Verseifung mit alkoholischer KOH und argentometrische Bestimmung des ionogenen Chlors),

daraus berechnet sich an nicht abspaltbarem Chlor: 10,20%.

Etwa $^1/_3$ des gesamten organisch gebundenen Chlors ist also für die Verseifung zum Aldehyd wertlos.

Ein sehr ähnliches Ergebnis wurde erhalten, als zur Chlorierung anstelle des Chlors die äquivalente Menge Sulfurylchlorid verwendet wurde.

## Patentansprüche

1. Verfahren zur Herstellung von p-tert.-Butylbenzaldehyd und dessen am Kern durch Halogen substituierten Derivaten,

dadurch gekennzeichnet dass man

a) p-tert.-Butyltoluol und dessen am Kern durch Halogen substituierte Derivate mit etwa 2 mol Brom/mol organisches Ausgangsmaterial bei Temperaturen von 40 bis 200°C, vorzugsweise 40 bis 120°C, gegebenenfalls unter der Einwirkung energiereicher Strahlung oder in Gegenwart von Radikalbildnern umsetzt und

b) das dabei gebildete p-tert. Butylbenzalbromid und dessen am Kern durch Halogen substituierte Derivate gegebenenfalls nach deren Isolierung bei erhöhter Temperatur, gegebenenfalls in Gegenwart von üblichen Verseifungskatalysatoren, mit Wasser verseift.

2. Verfahren nach Anspruch 1, dadurch gekennzeichnet, dass man als am Kern durch Halogen substituierte Derivate des p-tert.-Butyltoluols die monosubstituierten Derivate der Formel (I):

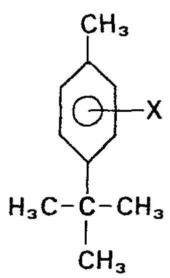

(I)

worin X = F, Cl, Br, J, insbesondere F, Cl, Br.

3. Verfahren nach Anspruch 1 bis 2, dadurch gekennzeichnet, dass man als energiereiche Strahlung UV-Licht verwendet.

4. Verfahren nach Anspruch 1 bis 3, dadurch gekennzeichnet, dass man die Verseifung in Stufe b bei Temperaturen von 60 bis 150°C, vorzugsweise von 80 bis 120°C, durchführt.

5. Verfahren nach Anspruch 1 bis 4, dadurch gekennzeichnet, dass man während oder nach der Verseifung noch eine geringe Menge $CH_2O+NH_3$ oder eine dieser $CH_2O$- und $NH_3$-Menge äquivalente Menge Hexamethylentetramin in wässriger Lösung zusetzt und in Gegenwart dieser Zusatzstoffe erhitzt.

6. Verfahren nach Anspruch 5, dadurch gekennzeichnet, dass man die Zusatzstoffe $CH_2O+NH_3$ oder das Hexamethylentetramin nach der Verseifung des p-tert.-Butylbenzalbromids oder von dessen am Kern durch Halogen substituierten Derivaten zusetzt und auf Temperaturen zwischen 60 bis 150°C, vorzugsweise 80 bis 120°C, erhitzt.

7. Verfahren nach Anspruch 5 bis 6, dadurch gekennzeichnet, dass man pro mol vorhandenes p-tert.-Butylbenzylbromid oder dessen am Kern durch Halogen substituiertes Derivat 1,1 bis 4 mol, vorzugsweise 1,1 bis 2 mol $CH_2$+die gleiche Menge $NH_3$ oder eine dieser $CH_2O$- und $NH_3$-Menge äquivalente Menge Hexamethylentetramin einsetzt.

## Claims

1. Process for the manufacture of p-tert.-butylbenzaldehyde and its derivatives halogen-substituted at the nucleus, characterized in

a) reacting p-tert.-butyltoluene and the derivatives thereof halogen-substituted at the nucleus with approximately 2 mol of bromine/mol of organic starting compound, at a temperature of from 40 to 200°C, preferably 40 to 120°C, optionally under the action of high energy radiation or in the presence of radical-forming agents, and

b) saponifying with water the p-tert.-butylbenzalbromide and the derivatives thereof halogen-substituted at the nucleus obtained, optionally after their isolation at elevated temperature, if desired in the presence of the usual saponification catalysts.

2. The process of claim 1, characterized in using as derivatives of p-tert.-butyltoluene halogen-substituted at the nucleus the monosubstituted derivatives of the formula I:

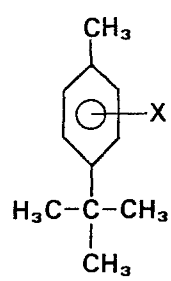

(I)

in which X denotes F, Cl, Br, I, preferably F, Cl, Br.

3. The process of claim 1 or 2, characterized in using as high energy radiation ultraviolet light.

4. Process of claims 1 to 3, characterized in carrying out the saponification of stage b at a temperature of from 60 to 150°C, preferably of from 80 to 120°C.

5. The process of claims 1 to 4, characterized in adding, during or after saponification, still a small amount of $CH_2+NH_3$ or an amount of hexamethylenetetramine equivalent to said $CH_2O+NH_3$ amount in aqueous solution, and heating in the presence of these substances.

6. The process of claim 5, characterized in

adding the $CH_2O+NH_3$ or the hexamethylenetetramine after the saponification of the p-tert.-butylbenzalbromide and the derivatives thereof halogen-substituted at the nucleus, and heating to a temperature of from 60 to 150°C, preferably of from 80 to 120°C.

7. The process of claims 5 to 6, characterized in using for each mol of p-tert.-butylbenzylbromide or its derivative halogen-substituted at the nucleus 1.1 to 4 mol, preferably 1.1 to 2 mol, of $CH_2O$+the same amount of $NH_3$ or an amount of hexamethylenetetramine equivalent to said $CH_2O+NH_3$ amount.

## Revendications

1. Procédé de préparation du p-tert.-butylbenzaldéhyde et de ses dérivés de substitution halogénés sur le noyau, procédé caractérisé en ce que:

a) on fait réagir le p-tert.-butyltoluène, ou ses dérivés de substitution halogénés sur le noyau, avec environ 2 mol de brome/mol de matière de départ organique, à des températures de 40 à 200°C, de préférence de 40 à 120°C, éventuellement sous l'action d'un rayonnement de haute énergie ou en présence de générateurs de radicaux, et

b) on saponifie par de l'eau le bromure de p-tert.-butylbenzylidène ainsi formé, ou ses dérivés halogénés sur le noyau, éventuellement après les avoir isolés, à température élevée, éventuellement en présence de catalyseurs de saponification usuels.

2. Procédé selon la revendication 1, caractérisé en ce qu'on utilise, comme dérivés du p-tert.-butyltoluène halogénés sur le noyau, les dérivés monosubstitués répondant à la formule (I):

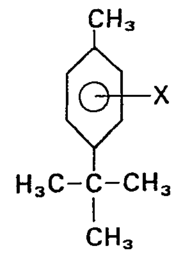

(I)

dans laquelle X représente F, Cl, Br ou I, plus spécialement F, Cl ou Br.

3. Procédé selon l'une des revendications 1 et 2, caractérisé en ce qu'on utilise un rayonnement ultraviolet comme rayonnement de haute énergie.

4. Procédé selon l'une quelconque des revendications 1 à 3, caractérisé en ce qu'on effectue la saponification du stade b à des températures comprises entre 60 et 150°C, de préférence entre 80 et 120°C.

5. Procédé selon l'une quelconque des revendications 1 à 4, caractérisé en ce qu'on ajoute, en outre, pendant ou après la saponification, une petite quantité de $CH_2O+NH_3$ ou une quantité d'hexaméthylènetétramine équivalent à cette quantité de $CH_2O+NH_3$, en solution aqueuse, et en ce qu'on chauffe en présence de ces additifs.

6. Procédé selon la revendication 5, caractérisé en ce qu'on ajoute les additifs $CH_2O+NH_3$ ou l'hexaméthylènetétramine après la saponification du bromure de p-tert.-butylbenzylidène, ou de ses dérivés halogénés sur le noyau, et en ce qu'on chauffe à des températures comprises entre 60 et 150°C, de préférence entre 80 et 120°C.

7. Procédé selon l'une des revendications 5 et 6, caractérisé en ce qu'on utilise, par mole de bromure de p-tert.-butylbenzyle présent, ou de son dérivé halogéné sur le noyau, de 1,1 à 4 mol, de préférence de 1,1 à 2 mol, de $CH_2O$+la même quantité de $NH_3$, ou une quantité d'hexaméthylènetétramine équivalent à cette quantité de $CH_2O+NH_3$.